# EUROPÄISCHE PATENTANMELDUNG

(11) **EP 1 342 463 A1**
(43) Veröffentlichungstag der Anmeldung: **10.09.2003**
(21) Anmeldenummer: 03004714.6
(22) Anmeldetag: 04.03.2003
(51) Int. Cl.: A61K 7/00

(54) **Lippenpflegeprodukte mit UV-Schutz**

(30) Priorität: 08.03.2002 DE 10210337
(71) Anmelder: Beiersdorf AG, 20245 Hamburg (DE)
(72) Erfinder: Göppel, Anja, 22527 Hamburg (DE); Schleyer, Karin, 20255 Hamburg (DE); Riedel, Cornelia, 21075 Hamburg (DE); Warnke, Katja, Dr., 20257 Hamburg (DE)

(57) **Zusammenfassung**

Geschmacksneutrale kosmetische und/oder dermatologische Sonnenschutzzubereitungen, Plegezubereitungen, Dekorationszubereitungen zum Auftragen auf die Lippen mit einem Gehalt an Bis-Ethylhexyloxyphenol Methoxyphenyl Triazin, weiteren UV-A- und UV-B-Filtern und polaren Ölen mit einer Polarität von höchstens 30 mN/m.

## Beschreibung

Die vorliegende Erfindung betrifft geschmacksneutrale Lippenpflegeprodukte mit einem Gehalt an Lichtschutzmitteln.

Lippenpflegeprodukte werden vor allem in Form von Stiften hergestellt, daneben gibt es aber auch Glosse, Creme- und Gelformulierungen.

Die meisten Stiftformulierungen sind wasserfreie Fettmischungen aus festen oder halbfesten Wachsen und flüssigen Ölen, wobei die hochgereinigten Paraffinöle und-wachse die Lippenstiftgrundmasse darstellen. Auch wasserhaltige Zubereitungen sind bekannt. Bei diesen ist der Wassergehalt im Allgemeinen sehr gering. Durch besondere Technologien sind aber auch Wassergehalte bis über 50 % erreichbar in Stiften. Übliche Grundstoffe für stiftförmige Zubereitungen sind beispielsweise flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) hochschmelzende Wachse (z.B. Carnaubawachs, Candelillawachs). Lippenstifte des Standes der Technik mit einem Gehalt an Paraffinen und Bienenwachs sind in "Kosmetik, Entwicklung Herstellung und Anwendung kosmetischer Mittel", S. 105, Herausgeber: W. Umbach, Georg Thieme Verlag, Stuttgart - New York, 1988, beschrieben.

Wegen der hohen Empfindlichkeit des Lippenbereiches, insbesondere gegenüber ultravioletter Strahlung infolge des praktisch völligen Mangels an Pigmenten, empfiehlt sich, zumal bei erhöhter UV-Exposition wie im Hochgebirge, dem Lippenbereich einen Schutz gegen UV-Strahlung in Form von entsprechenden Lichtschutzzubereitungen zukommen zulassen. Gerade in stiftförmigen Zubereitungen des Standes der Technik werden oft anorganischen Pigmente als UV-Absorber bzw. UV-Reflektoren zum Schütze des Lippenbereiches vor UV-Strahlen verwendet. Dabei handelt es sich insbesondere um Oxide des Titans, aber auch gelegentlich des Zinks, Eisens, Zirkoniums, Siliciums, Mangans, Aluminiums, Cers und Mischungen davon, sowie Abwandlungen. Daneben ist oft der Einsatz von Lichtschutzmitteln in Lippenpflegeprodukten erforderlich, um die Lippen effektiv vor UV-Strahlung zu schützen. Dabei ist der Geschmack der Formulierung im Gegensatz zu anderen Lichtschutzmittel von wesentlicher Bedeutung für den Anwender. Herkömmliche Lichtschutzfilter wie Butyl-Methoxydibenzoylmethan und Methylbenzyliden Campher als UV-Filter weisen einen extrem bitteren Geschmack auf und sind daher nur begrenzt einsetzbar. Der Anwender toleriert den unangenehmen Eigensgeschmack derartiger Formulierungen nur dann, wenn er auf einen hohen UV-Schutz Wert legt. Wünschenswert wäre es, geschmacksneutrale Lippenpflegeprodukte mit hohen UV-A und UV-B Lichtschutzfaktoren anbieten zu können.

Zur Lösung dieses Problems gibt es verschiedene Ansätze. Eine Möglichkeit besteht darin, den unangenehmen Eigengeschmack der Filtersubstanz durch Geruchsstoffe zu überdecken. Dies gelingt besonders gut, wenn diese auch als geschmacklich wahrnehmbar sind. Eine andere Möglichkeit ist der Einsatz von Aromastoffen oder auch von Süßstoffen, die vom geschmacklichen Eindruck der Lichtfilter ablenken. Auch durch Auswahl der Lipidgrundlage kann der geschmackliche Eindruck vorteilhaft beeinflußt werden. Eine weitere Möglichkeit besteht im Einsatz von Lichtschutzmitteln auf Basis anorganischer Pigmente, die naturgemäß keinen Eigengeschmack haben. Damit lassen sich jedoch nur geringe Lichtschutzfaktoren realisieren, ohne bei Anwendung des Produktes einen störenden weißen Farbeindruck auf den Lippen zu erzeugen.

Es war nach all diesem überraschend und nicht vorhersehbar, daß geschmacksneutrale kosmetische und/oder dermatologische Sonnenschutzzubereitungen, Plegezubereitungen, Dekorationszubereitungen zum Auftragen auf die Lippen mit einem Gehalt an Bis-Ethylhexyloxyphenol Methoxyphenyl Triazin, weiteren UV-A- und UV-B-Filtern und polaren Ölen mit einer Polarität von höchstens 30 mN/m den Nachteilen des Standes der Techniik abhelfen. Dabei ist es bevorzugt, wenn diese Zubereitungen in Form von Stiften, Gelen, W/O und O/W-Emulsionen sowie Glossen vorliegen.

Bevorzugt werden die polaren Ölen gewählt aus folgender Liste:
- native Lipide, bevorzugt Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl,
- synthetische Lipide, bevorzugt Isodecyl Neopentanoate, Isohexyldecanoate, Isodecyl Octanoate, Dihexyl Ether, Isodecyl-3,5,5-Trimethyl Hexanoate, Cetearyl Isononanoate, Isopropylpalmitat, Cyclomethicon, Cyclopolydimethylsiloxan, Dimethicon, 2- Ethylhexanosäure-3,5,5-Trimethylester, Octyldodecanol, Hexyl Decanol, Isotridecyl-3,5,5-Trimethylhexanonanoat, Hexyldecanol (+) Hexyl Decyl Laurat, Octyl Palmitat, Octyldodeceyl Myristat, Phenyl Trimethicon, Butyl OctanoicacidIsopropyl Stearat, C12-15 Alkyl Benzoat, Butylen Glycol Caprylat/Caprat, Caprylic/Capric Triglycerid, Tricaprylin, Diethylhexanoat/Diisononanoat/Ethylhexyl Isononanoat, Ethylhexyl Isononanoat, Propylene Glycol Dicaprylat/Dicaprat, Butyl Octanol, Stearyl Heptanoat, Dibutyl Adipat, PEG 2 Diethylenhexanoat, C12-13 Alkyl Lactat, Di-C-12/13 Alkyl Tartrat, Propylen Glycol Monoisostearat, Cocoglyceride, Triisostearin,
- Butyl Decanol (+) Hexyl Octanol (+), Hexyl Decanol (+) Butyl Octanol, Guerbet-Alkohol-Fettsäureestere, Propylenglycoldiester der Capryl/Caprinsäure, Tridecyl Stearat (+) Tridecyl Trimellitat (+) Dipentaerythrityl Hexacaprylat/Hexacaprat Diethylen Glycol Dioctanoat(/ Diisononanoat),
- Paraffinöl und/oder hydrierte Polyolefine, bevorzugt hydriertes Polyisobuten, Squalan und/oder Squalen,
- bei Raumtemperatur flüssige UV-Filtersubstanzen, bevorzugt Homomenthylsalicylat, 2-Ethylhexyl-2-cyano-3,3-diphenylacrylat, 2-Ethylhexyl-2-hydroxybenzoat und Ester der Zimtsäure, bevorzugt 4-Methoxyzimtsäure(2-ethylhexyl)ester und 4-Methoxyzimtsäureisopentylester.

Weiterhin ist es bevorzugt, wenn die Zubereitungen einen Gehalt an Bisethylhexyloxyphenolmethoxyphenyltriazin und mindestens einer weitern aus folgender Liste gewählten UV-Filtersubstanz:
Homomenthylsalicylat, 2-Ethylhexyl-2-cyano-3,3-diphenylacrylat, 2-Ethylhexyl-2-hydroxybenzoat, 2-(4'-(Diethylamino)-2'-hydoxybenzoyl)-benzoesäurehexylester und
Ester der Zimtsäure, bevorzugt 4-Methoxyzimtsäure(2-ethylhexyl)ester und/oder 4-Methoxyzimtsäureisopentylester aufweisen.

Ebenso ist es bevorzugt, wenn diese Zubereitungen einen Gehalt an anorganischen Pigmenten gewählt aus der Gruppe der Metalloxide und/oder anderen in Wasser schwerlöslichen oder unlösliche Metallverbindungen, bevorzugt Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄) aufweisen.

Bei all diesem ist es bevorzugt, wenn der Gehalt an UV-Filtersubstanzen 0,1 Gew.-% bis 20 Gew.-%, besonders bevorzugt 0,5 bis 15 Gew.-%, ganz besonders bevorzugt 1,0 bis 12,0 Gew.-%, jeweils bezogen auf das Gesamtgewicht der Zubereitungen, beträgt.

Dabei ist es besonders bevorzugt, wenn der Gehalt an 2-Ethylhexyl-2-cyano-3,3-diphenylacrylat höchstens 4 Gew.%, Homomenthylsalicylat höchstens 10 Gew.%, 2-Ethylhexyl-2-hydroxybenzoat höchstens 5 Gew.%, 4-Methoxyzimtsäure(2-ethylhexyl)ester höchstens 10 Gew.%, 4-Methoxyzimtsäureisopentylester höchstens 5 Gew.%, Phenylen-1,4-bis-(2-benzimidazyl)-3,3'-5,5'-tetrasulfonsäure-bis-natriumsalz höchstens 10 Gew.%, 2-Phenylbenzimidazol-5-sulfonsäure höchstens 8 Gew.%, Diethylhexylbutylamidotriazon höchstens 10 Gew.%, 4,4',4"-(1,3,5-Triazin-2,4,6-triyltriimino)-tris-benzoësäure-tris(2-ethylhexylester) höchstens 5 Gew.% beträgt.

Ebenso ist es bevorzugt, wenn der Gehalt an polaren Ölen 5 Gew.% bis 30 Gew.-%, besonders bevorzugt 7 bis 20 Gew.-%, ganz besonders bevorzugt 10 bis 15,0 Gew.-%, jeweils bezogen auf das Gesamtgewicht der Zubereitungen, beträgt.

Derartige Zubereitungen werden bevorzugt verwendet zur Herstellung von geschmacksneutralen Lippenpflegezubereitungen, Lippendekorationszubereitungen und/oder Sonnenschutzzubereitungen zum Auftragen auf die Lippen.

Es ist von Vorteil, wenn diese Zubereitungen Bis-Ethylhexyloxyphenol Methoxyphenyl Triazin als alleinigen organischen UV-Filter enthalten.
Die anorganischen Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 | Octyltrimethylsilan | Degussa |

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Diethylhexylbutylamidotriazon (INCI: Diethylhexylbutamidotriazone), welches unter der Handelsbezeichnung UVASORB HEB bei Sigma 3V erhältlich ist;
- 4;4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Die beschriebenen Lichtschutzformulierungen können vorteilhaft in Form von Fettstiften mit höchstens geringem Wassergehalt, in Form von W/O-Emulsionen und auch O/W-Emulsionen als Stift oder Creme und als Gel oder Gloss eingesetzt werden.
Außerdem können die erfindungsgemäßen Stifte, Gel, Glosse und Emulsionen Farbstoffe und/oder Pigmente enthalten, insbesondere wenn sie in Form von dekorativen Lippenstiften, Lippenkonturenstiften, Abdeckstiften und/oder Kombistiften für Lippe/Gesicht/Auge vorliegen.

Die erfindungsgemäßen Emulsionen können Farbstoffe und/oder Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.

Pigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische Pigmente bestehen aus unlöslichen Salzen von zertifizierten Farbstoffen, die als Lakes oder Eisenoxide bezeichnet werden. Beispielsweise können Barium lakes, calcium lakes, aluminum lakes, titandioxide, mica and iron oxides Verwendung finden. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake, Blue 1 Aluminum Lake und Kombinationen einsetzbar.

Als Eisenoxide oder -oxidhydrate sind z.B. cosmetic yellow oxide C22-8073 (Sunchemical) cosmetic oxide MC 33-120 (Sunchemical), cosmetic brown oxide C33-115 (Nordmann& Rassmann), cosmetic russet oxide C33-8075 (Sunchemical) bekannt und gegebenfalls vorteilhaft. Als Alumosilicat ist ultramarinblau (Les colorants Wacker) einsetzbar.

Auch Perlglanzpigmente lassen sich in die erfindungsgemäßen Emulsionen einarbeiten. Diese sind beipsielsweise von den Firmen Costenoble (Cloisonne-Typ, Flamenco-Typ, Low Lustre-Typ), Merck (Colorona-Typen, Microna-Typ, Timiron-Typ, Colorona, Ronasphere), Les Colornats Wacker (Covapure, Vert oxyde de Chrome), Cadre (Colorona, Sicopearl), BASF (Sicopearl, Sicovit), Rona (Colorona) bekannt. Als besonders vorteilhaftes Perlglanzpigment haben sich beispielsweise Timiron Silk Gold und Colrona Red Gold bewährt.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour index, 3. Auflage, Society of Dyers and Colourists, Bradford, England, 1971* entnommen.

Vorteilhafte Koponenten sind die mit der CIN 10006, 10020, 10316, 11680, 11710, 11725, 11920, 12010, 12085, 12120, 12150, 12370, 12420, 12480, 12490, 12700, 13015, 14270, 14700, 14720, 14815, 15510, 15525, 15580, 15620, 15630, 15800, 15850, 15865, 15880, 15980, 15985, 16035, 16185, 16230, 16255, 16290, 17200, 18050, 18130, 18690, 18736, 18820, 18965, 19140, 20040, 20170, 20470, 21100, 21108, 21230, 24790, 27290, 27755, 28440, 40215, 40800, 40820, 40825, 40850, 42045, 42051, 42053, 42080, 42090, 42100, 42170, 42510, 42520, 42735, 44045, 44090, 45100, 45190, 45220, 45350, 45370, 45380, 45396, 45405, 45410, 45425, 45430, 47000, 47005, 50325, 50420, 51319, 58000, 59040, 60724, 60725, 60730, 61565, 61570, 61585, 62045, 69800, 69825, 71105, 73000, 73015, 73360, 73385, 73900, 73915, 74100, 74160, 74180, 74260, 75100,75120, 75125, 75130, 75135, 75170, 75300, 75470, 75810, 77000, 77002, 77004, 77007, 77015, 77120, 77163, 77220, 77231, 77266, 77267, 77268:1, 77288, 77289, 77346, 77400, 77480, 77489, 77491, 77492, 77499, 77510, 77713, 77742, 77745, 77820, 77891, 77947 sowie 6,7-Dimethyl-9-(1'-D-ribityl)-isoalloxazin und Lactoflavin, Zuckerkulör, Capsanthin, Capsorubin, Betanin, Benzopyryliumsalze, Anthocyane, Aluminium-, Zink-, Magnesium- und Calciumstearat, Bromthymolblau, Bromkresolgrün, Acid Red 195.

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalondisulfosäure, Aluminiumsalz der Indigo-disulfosäure, rotes und schwarzes Eisenoxid (CIN: 77 491 (rot) und 77499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Die Liste der genannten Farbstoffe und Farbpigmente, die in den erfindungsgemäßen Lippenprodukten verwendet werden können, soll selbstverständlich nicht limitierend sein.

Erfindungsgemäß können auch lipophilen Wirkstoffe sehr vorteilhaft in die Lippenprodukte mit UV-Schutz eingearbeitet werden. Insbesondere geeignet sind:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl®, Eucerit® und Neocerit®.

Es ist gegebenenfalls möglich und vorteilhaft, die erfindungsgemäßen Zubereitungen als Grundlage für pharmazeutische Formulierungen und/oder parfümfreie Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.
Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Die nachfolgenden Beispiele sollen die Erfindung erläutern, ohne sie einzuschränken.

Alle Mengenangaben; Prozentangaben oder Teile beziehen sich, soweit nicht anders angegeben, auf das Gewicht, insbesondere auf das Gesamtgewicht der Zubereitungen oder der jeweiligen Mischungen.

### Beispiele

| **Gloss** | **1** |
|---|---|
| Polyisobuten | ad. 100 |
| Pentaerythrityl Tetraisostearat | 8,0 |
| Myristyl Lactat | 6,0 |
| Mica + 77891 | 3,5 |
| Macadamianussöl (Macadamia Ternifolia) | 3,0 |
| Disteardimonium Hectorit | 2,0 |
| Siliciumdioxid (Silica) | 2,0 |
| Mica + Cl 77891 (Mica + Titandioxide ) | 1,4 |
| Bis-Ethylhexyloxyphenol Methoxyphenyl Triazin | 1,0 |
| Tocopheryl Acetat | 1,0 |
| Cl 77491 + Mica + Cl 77891 (Eisenoxide + Mica + Titandioxid ) | 1,0 |
| Propylparaben | 0,4 |
| Parfüm | 0,2 |
| BHT | 0,05 |

| **Lippenpflegestifte** | **2** | **3** | **4** |
|---|---|---|---|
| Caprylic/Capric Triglyceride | 20 | 10 | 7 |
| Octyldodecanol | 20 | 7 | 7 |
| Pentaerythrityl Tetraisostearat | | 10 | 9 |
| Polyglyceryl-3 Diisostearat | 3,7 | 2,5 | 2,5 |
| Bis-Diglyceryl Polyacyladipat-2 | | 9 | 9 |
| Cetearylalkohol | 6 | 10 | 8 |
| Myristyl Myristat | 7,5 | 3,5 | 3,5 |
| C16-36 Alkylstearat | | 17 | 15 |
| Stearyl Bienenwachs + Behenyl Bienenwachs | | 5 | 5 |
| C18-38 Alkyl Hydroxystearoyl Stearat | | 2 | 2 |
| Cera Carnauba | | 1,5 | 1,5 |
| Cera Alba | 6 | 0,5 | 0,5 |
| C20-40 Alkylstearat | 16 | | 2 |
| Titandioxid micronisiert | | | 1 |
| Ethylhexyl Methoxycinnamat | 3 | 3 | |
| Bis-Ethylhexyloxyphenol Methoxyphenyl Triazin | 0,25 | 1 | 4 |
| Octocrylen | | | 4 |
| Mica | | | 1,5 |
| Farbpigmente Cl 77891, 42090, 73360, 19140, 15985, 45380, 45410, 15850, 47005, 40800 | q.s | q.s | 10 |
| Interferenzpigmente mit und ohne Farbe | | | 1,5 |
| Tocopheryl Acetat | 1 | 0,5 | 1 |
| Bisabolol | | 1 1 | 1 |
| Tocopherol;Lecithin ;Ascorbyl Palmitat | 0,05 | 0,05 | 0,05 |
| Jojobaöl (Buxus Chinensis) | 6 | 2 | 1 |
| Lanolinalkohol | 0,2 | | |
| Mandelöl (Prunus Dulcis) | | | 1 |
| Avocadoöl (Persea Gratissima) | | | 1 |
| Stearyl Glycyrrhetinat | | | 0,01 |
| Allantoin | | | 0,1 |
| Natriumhydroxid | 0,04 | | |
| Diammoniumcitrat | 0,08 | | |
| Wasser | 2,5 | | |
| Zitronensäure | 0,05 | | |
| Panthenol | | 0,125 | |
| Parfüm, BHT | q.s | q.s | q.s |
| Ricinusöl | ad.100 | ad.100 | ad.100 |

| **Lippenstift** | **5** |
|---|---|
| Ricinusöl | ad |
| Cl (77491, 77492, 77499, 77891, 77120, 42090, 17200) | 10,0 |
| Octyldodecanol | 9,0 |
| Hydrogeniertes Polydecen | 9,0 |
| Myristyl Lactat | 8,0 |
| Lanolinöl | 7,0 |
| Cera Microcristallina | 7,0 |
| Mica | 6,0 |
| Candelilla Cera | 6,0 |
| Bis-Diglyceryl Polyacyladipat-2 | 4,0 |
| Avocadoöl (Persea Gratissima) | 3,0 |
| Lauryl PCA | 3,0 |
| Cera Carnauba | 2,5 |
| Jojobaöl (Buxus Chinensis) | 2,0 |
| PVP/Hexadecen Copolymer | 2,0 |
| Lanolinalkohol | 2,0 |
| Bis-Ethylhexyloxyphenol Methoxyphenyl Triazin | 2,0 |
| Tocopherylacetat | 1,5 |
| Ethylhexylcrylen | 1,0 |
| Nylon-12 | 1,0 |
| Lecithin | 0,9 |
| Disteardimonium Hectorit | 0,8 |
| Parfüm | 0,5 |
| Propylencarbonat | 0,28 |
| Retinylpalmitat | 0,2 |
| Propylparaben | 0,1 |
| Ubiquinon | 0,1 |
| Biotin | 0,06 |
| BHT | 0,06 |

| **O/W Emulsion** | 6 |
|---|---|
| Aqua | Ad 100 |
| Glycerin | 9,0 |
| Cyclomethicon | 6,0 |
| Alcohol | 4,0 |
| Caprylic/Capric Triglycerid | 2,5 |
| Octyldodecanol | 2,5 |
| Glyceryl Stearate Citrat | 2,0 |
| Ethylhexyl Methoxicinnamate | 2,0 |
| Myristyl Myristat | 1,0 |
| Parabene | 0,7 |
| SodiumCarbomer | 0,5 |
| Cetearyl Alcohol | 0,5 |
| Bis-Ethylhexyloxyphenol Methoxyphenyl Triazin | 0,25 |
| Perfume | 0,2 |
| Summe | 100,0 |

| **Wasserhaltige Lippenstifte** | **7** | **8** | **9** |
|---|---|---|---|
| Mikrokristallines Wachs | | 4,5 | 4 |
| Ozokerit | | | 0,5 |
| Carnaubawachs | | 1,5 | 2,1 |
| C20-40- Alkylstearat | 8,0 | | |
| Candelillawachs | | 4,0 | 2,0 |
| PEG-8 Bienenwachs | 1,0 | | |
| Bienenwachs | | | 1,0 |
| PEG-45/ Dodecyl Glycol Copolymer | 1,6 | | |
| Polyglyceryl-3-diisostearat | 2,4 | | |
| Lauryl Pyrrolidoncarbonsäure | | | 3,0 |
| Lanolinöl | | 4,0 | |
| Bis-Diglyceryl Polyacyladipat-2 | | 3,5 | 5,0 |
| Simethicon | 0,5 | 1,0 | |
| Isopropyl Palmitat | | 3,5 | 4,0 |
| Squalan | 1,0 | | |
| Triisostearin | | 3,0 | |
| Myristyl Lactat | | 4,0 | |
| Jojobaöl | 1,0 | 2,0 | |
| Caprylic/Capric Triglycerid | | | 5,0 |

| Ethylhexyl Cocoat | 4,8 | | |
|---|---|---|---|
| Pentaerythrityl Tetraisostearat | | | 1,0 |
| Isodecyl Neopentanoat | | | 2,0 |
| Dicaprylyl Carbonat | | | 2,0 |
| Hydrogeniertes Polydecen | 4,8 | 2,5 | |
| Octyldodecanol | 4,0 | 2,5 | |
| PVP/Hexadecen Copolymer | | | 0,5 |
| Ethylhexyl Methoxycinnamat | 2,0 | | 0,5 |
| Bis-Ethylhexyloxyphenol Methoxyphenyl Triazin | 0,5 | 1,0 | 0,5 |
| Mikronisiertes Titandioxid (Eusolex T 2000) | | 2,0 | 4,0 |
| Silica Dimethyl Silylat (Aerosil R972) | | | 0,3 |
| Polymethylsilsesquioxan (Tospearl 2000B) | | | 0,3 |
| Interferenz Pigmente | | | 4,0 |
| Titandioxid Cl 77891 | 2,5 | 4,0 | 3,8 |
| Eisenoxide Cl 77491, 77492, 77499 | 1,8 | 3,2 | 2,2 |
| D&C Rot 7 | 0,5 | 0,6 | |
| D&C Rot 33 | | | 0,3 |
| FD&C Gelb 6 | | | 1,5 |
| D&C Rot 28 | 1,0 | | |
| D&C Rot 34 | 0,2 | | |
| Tocopheryl Acetat | | 1,0 | |
| Ubichinon | 0,001 | 0,001 | 0,001 |
| Xylitol | | 2,0 | |
| Glycerin | 10,0 | 5,0 | 3,0 |
| Konservierungsmittel, BHT, Parfum, Aroma | q.s. | q.s. | q.s. |
| Wasser | ad 100 | 30,0 | 40,0 |
| Ricinusöl | 5,0 | ad 100 | ad 100 |

## Patentansprüche

1. Geschmacksneutrale kosmetische und/oder dermatolögische Sonnenschutzzubereitungen, Plegezubereitungen, Dekorationszubereitungen zum Auftragen auf die Lippen mit einem Gehalt an Bis-Ethylhexyloxyphenol Methoxyphenyl Triazin, weiteren UV-A- und UV-B-Filtern und polaren Ölen mit einer Polarität von höchstens 30 mN/m.

2. Kosmetische und/oder dermatologische Zubereitungen nach Anspruch 1 in Form von Stiften, Gelen, W/O und O/W-Emulsionen sowie Glossen.

3. Kosmetische und/oder dermatologische Zubereitungen nach mindestens einem der vorangehenden Ansprüche **gekennzeichnet durch** einen Gehalt an polaren Ölen gewählt aus folgender Liste:
• native Lipide, bevorzugt Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl,
• synthetische Lipide, bevorzugt Isodecyl Neopentanoate, Isohexyldecanoate, Isodecyl Octanoate, Dihexyl Ether, Isodecyl-3,5,5-Trimethyl Hexanoate, Cetearyl Isononanoate, Isopropylpalmitat, Cyclomethicon, Cyclopolydimethylsiloxan, Dimethicon, 2- Ethylhexanosäure-3,5,5-Trimethylester, Octyldodecanol, Hexyl Decanol, lsotridecyl-3,5,5-Trimethylhexanonanoat, Hexyldecanol (+) Hexyl Decyl Laurat, Octyl Palmitat, Octyldodeceyl Myristat, Phenyl Trimethicon, Butyl OctanoicacidIsopropyl Stearat, C12-15 Alkyl Benzoat, Butylen Glycol Caprylat/Caprat, Caprylic/Capric Triglycerid, Tricaprylin, Diethylhexanoat/Diisononanoat/Ethylhexyl Isononanoat, Ethylhexyl Isononanoat, Propylene Glycol Dicaprylat/Dicaprat, Butyl Octanol, Stearyl Heptanoat, Dibutyl Adipat, PEG 2 Diethylenhexanoat, C12-13 Alkyl Lactat, Di-C12/13 Alkyl Tartrat, Propylen Glycol Monoisostearat, Cocoglyceride, Triisostearin,
• Butyl Decanol (+) Hexyl Octanol (+), Hexyl Decanol (+) Butyl Octanol, Guerbet-Alkohol-Fettsäureestere, Propylenglycoldiester der Capryl/Caprinsäure, Tridecyl Stearat (+) Tridecyl Trimellitat (+) Dipentaerythrityl Hexacaprylat/Hexacaprat Diethylen Glycol Dioctanoat(/ Diisononanoat),
• Paraffinöl und/oder hydrierte Polyolefine, bevorzugt hydriertes Polyisobuten, Squalan und/oder Squalen,
• bei Raumtemperatur flüssige UV-Filtersubstanzen, bevorzugt Homomenthylsalicylat, 2-Ethylhexyl-2-cyano-3,3-diphenylacrylat, 2-Ethylhexyl-2-hydroxybenzoat und Ester der Zimtsäure, bevorzugt 4-Methoxyzimtsäure(2-ethylhexyl)ester und 4-Methoxyzimtsäureisopentylester.

4. Kosmetische und/oder dermatologische Zubereitungen nach mindestens einem der vorangehenden Ansprüche **gekennzeichnet durch** einen Gehalt an Bisethylhexyloxyphenolmethoxyphenyltriazin und mindestens einer weitern aus folgender Liste gewählten UV-Filtersubstanz:
Homomenthylsalicylat, 2-Ethylhexyl-2-cyano-3,3-diphenylacrylat, 2-Ethylhexyl-2-hydroxybenzoat, 2-(4'-(Diethylamino)-2'-hydoxybenzoyl)-benzoesäurehexylester und
Ester der Zimtsäure, bevorzugt 4-Methoxyzimtsäure(2-ethylhexyl)ester und/oder 4-Methoxyzimtsäureisopentylester.

5. Kosmetische und/oder dermatologische Zubereitungen nach mindestens einem der vorangehenden Ansprüche **gekennzeichnet durch** einen Gehalt an anorganischen Pigmenten gewählt aus der Gruppe der Metalloxide und/oder anderen in Wasser schwerlöslichen oder unlösliche Metallverbindungen, bevorzugt Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

6. Kosmetische und/oder dermatologische Zubereitungen nach mindestens einem der vorangehenden Ansprüche **dadurch gekennzeichnet, daß** der Gehalt an UV-Filtersubstanzen 0,1 Gew.-% bis 20 Gew.-%, bevorzugt 0,5 bis 15 Gew.-%, besonders bevorzugt 1,0 bis 12,0 Gew.-%, jeweils bezogen auf das Gesamtgewicht der Zubereitungen, beträgt.

7. Kosmetische und/oder dermatologische Zubereitungen nach mindestens einem der vorangehenden Ansprüche **dadurch gekennzeichnet, daß** der Gehalt an 2-Ethylhexyl-2-cyano-3,3-diphenylacrylat höchstens 4 Gew.%, Homomenthylsalicylat höchstens 10 Gew.%, 2-Ethylhexyl-2-hydroxybenzoat höchstens 5 Gew.%, 4-Methoxyzimtsäure(2-ethylhexyl)ester höchstens 10 Gew.%, 4-Methoxyzimtsäureisopentylester höchstens 5 Gew.%, Phenylen-1,4-bis-(2-benzimidazyl)-3,3'-5,5'-tetrasulfonsäure-bis-natriumsalz höchstens 10 Gew.%, 2-Phenylbenzimidazol-5-sulfonsäure höchstens 8 Gew.%, Diethylhexylbutylamidotriazon höchstens 10 Gew.%, 4,4',4"-(1,3,5-Triazin-2,4,6-triyltriimino)-tris-benzoësäure-tris(2-ethylhexylester) höchstens 5 Gew.% beträgt.

8. Kosmetische und/oder dermatologische Zubereitung nach mindestens einem der vorangehenden Ansprüche **dadurch gekennzeichnet, daß** der Gehalt an polaren Ölen 5 Gew.% bis 30 Gew.-%, vorzugsweise 7 bis 20 Gew.-%, besonders bevorzugt 10 bis 15,0 Gew.-%, jeweils bezogen auf das Gesamtgewicht der Zubereitungen, beträgt.

9. Verwendung von Zubereitungen nach einem der vorangehenden Ansprüche zur Herstellung von geschmacksneutralen Lippenpflegezubereitungen, Lippendekorationszubereitungen und/oder Sonnenschutzzubereitungen zum Auftragen auf die Lippen.
